(19) Europäisches Patentamt
European Patent Office
Office européen des brevets

(11) Veröffentlichungsnummer: **0 052 076**
**B1**

# (12) EUROPÄISCHE PATENTSCHRIFT

(45) Veröffentlichungstag der Patentschrift: **29.05.85**

(51) Int. Cl.⁴: **A 61 K 9/32**, A 61 K 9/36, A 61 K 9/52

(21) Anmeldenummer: **81810444.0**

(22) Anmeldetag: **06.11.81**

## (54) Schnellzerfallende Arzneimittel-Presslinge.

(30) Priorität: **12.11.80 CH 8393/80**

(43) Veröffentlichungstag der Anmeldung: **19.05.82 Patentblatt 82/20**

(45) Bekanntmachung des Hinweises auf die Patenterteilung: **29.05.85 Patentblatt 85/22**

(84) Benannte Vertragsstaaten: **AT BE CH DE FR IT LI LU NL SE**

(56) Entgegenhaltungen:
**DE - A - 2 030 501**
**DE - A - 2 336 218**
**US - A - 4 140 756**
**US - A - 4 143 129**

**CHEMICAL ABSTRACTS, Band 82, Nr. 10, 10. März 1975, Seite 469, Nr. 64453a Columbus, Ohio, U.S.A. K. LECHMANN et al.: "Use of aqueous dispersions of synthetic materials to coat drug forms"**
**UNLISTED DRUGS, Band 31, Nr. 9, September 1979, Verbindung Nr. 133q**
**UNLISTED DRUGS, Band 31, Nr. 4, April 1979, Verbindung Nr. 52C**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Postfach, CH-4002 Basel (CH)**

(72) Erfinder: **Kopf, Helmut, Dr., Waldhofstrasse 10, CH-4310 Rheinfelden (CH)**

**EP 0 052 076 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft schnellzerfallende Arzneimittel-Preßlinge, die Arzneimittel in körniger Retardform enthalten.

Es ist bekannt, Arzneimittelwirkstoffe mit Wirkstoffabgabe-verzögernden Hilfsstoffen zu vermischen bzw. zu umhüllen und zu Körnern zu verarbeiten, die dann in dieser körnigen Form direkt verabreicht werden können, oder nach dem Abfüllen in Kapseln, oder erst nach Weiterverarbeitung zu Tabletten zur Therapie verwendet werden. Bisher bekannte gekörnte Retard-Arzneimittel hatten mannigfache Nachteile. Es ergaben sich Schwierigkeiten im Zusammenhang mit der Herstellung. Entweder war die Herstellung umständlich oder es war die Verwendung von organischen Lösungsmitteln notwendig oder es waren die Hilfsstoffe nicht ideal im Hinblick auf den anvisierten Effekt, nämlich die richtig verzögerte Wirkstoffabgabe. Schwierigkeiten ergaben sich auch mit den äußeren Eigenschaften dieser Körner, also z. B. mit der Freifließ-Fähigkeit oder der Feuchtigkeitsempfindlichkeit, Eigenschaften, die entweder bei der direkten Verabreichung, also bei der Dosierung und eventuell bei gleichzeitiger Einnahme mit Nahrungs- oder Genußmitteln oder bei der Abfüllung in Kapseln unangenehm in Erscheinung traten. Überdies sind Einzeldosen von über 600 mg in Kapseln verpackt nicht zweckmäßig, da die Kapseln zum Schlucken zu groß sind.

Die erfindungsgemäßen schnellzerfallenden Arzneimittel-Preßlinge sind dadurch gekennzeichnet, daß sie im wesentlichen aus einem verpreßten Gemisch von

a) einem Arzneimittel in körniger Retardform, bestehend aus einem granulierten oder kristallinen Arzneimittelwirkstoff, der umhüllt ist mit einem Hüllstoff, der im wesentlichen aus einem homogenen Gemisch eines wasserlöslichen oder in Wasser dispergierbaren Poly(H + meth)-acrylsäure(methyl + äthyl)esters und einer im Wasser unlöslichen, aber in Wasser dispergierbaren Äthylcellulose besteht, wobei das Gewichtsverhältnis 2,5 : 1 bis 5 : 1 beträgt, und

b) quervernetztem Polyvinylpyrrolidon als Sprengmittel mit hoher Sprengkraft sowie Bindemitteleigenschaften

bestehen.

Arzneimittel in körniger Retardform sind als solche bekannt, insbesondere als umhüllte Körner oder in Matrixform, also z. B. auch an Ionenaustauschern absorbiert. Dieser Stand der Technik wird z. B. durch DE-A-2 336 218, DE-A-2 030 501 und Chem. Abstr. 82, 64453a (1975) illustriert. Andererseits sind z. B. aus US-A-4 143 129 Tablettensprengmittel wie Polyvinylpyrrolidon bekannt.

Die beiden erfindungsgemäß verwendeten Hüllstoffe sind einzeln als solche bekannt. Sie eignen sich alleine angewandt für den Zweck der vorliegenden Erfindung aber nicht. Der erstgenannte ist sehr thermoplastisch und damit hergestellte umhüllte Körner neigen zum Verkleben. Der zweitgenannte andererseits ergibt in gebräuchlichen Mengen und Verarbeitungsverfahren bei der vorliegenden Anwendung eine zu wenig wirksame retardierende Umhüllung. Es konnte somit nicht erwartet werden, daß die Kombination der für den vorliegenden Zweck einzeln nicht geeigneten Hüllstoffe ein in jeder Beziehung sehr gutes Resultat ergibt. So sind die zunächst hergestellten Arzneimittelkörner freifließend, unempfindlich gegen Feuchtigkeit, geschmacksneutral und sie erzielen die gewünschte verzögerte Wirkstoffabgabe mit großer Gleichmäßigkeit. Durch mikroskopische Untersuchung wurde außerdem festgestellt, daß das einzelne Wirkstoffkorn sehr gleichmäßig überzogen ist, so daß es im wesentlichen seine ursprüngliche Form beibehält. Somit kann mit homogenem Ausgangsmaterial leicht ein homogenes Zwischenprodukt erzielt werden.

Als Wirkstoffe für die erfindungsgemäße körnige Arzneimittel-Retardform eignen sich besonders körnige oder kristalline Stoffe. Besonders geeignet sind feste Körner oder Monokristalle im Größenbereich 0,3—2 mm (Durchmesser), die eine gewisse mechanische Festigkeit aufweisen, was von besonderer Bedeutung ist bei der anschließenden Verarbeitung zu Preßlingen.

Als Hüllstoffe verwendet man einerseits Polyacrylsäureester der Formel

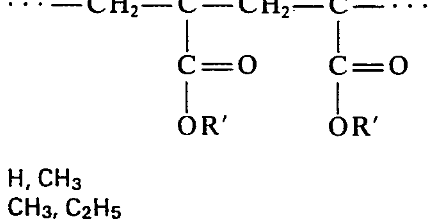

R = H, $CH_3$
R' = $CH_3$, $C_2H_5$

Solche Stoffe werden durch Emulsionspolymerisation gewonnen und enthalten das Copolymerisat mit einem Molgewicht von einigen 100 000 in Form von Latexteilchen mit einem Durchmesser um oder unter 1 µm. Ein entsprechendes, besonders geeignetes Produkt wird durch Röhm Pharma GmbH,

Darmstadt (BRD) unter der Bezeichnung Eudragit® E30D als wäßrige Dispersion vertrieben, und stellt ein Acrylsäureäthylester-Methacrylsäuremethylester-70 : 30-Copolymerisat mit Mol-Gew. 800 000 dar.

Andererseits wird als Hüllstoff Äthylcellulose verwendet. Besonders eignet sich ein Produkt, das von FMC Corporation, Philadelphia (Pennsylvania, USA) unter der Bezeichnung Aquacoat® ECD-30 vertrieben wird, und zwar als 30%ige wäßrige polymere Dispersion mit geringer Teilchengröße (Latexform) und enger Teilchengrößenverteilung.

Die beiden eben genannten Hüllstoffe [Poly(H+meth)-acrylsäure(methyl+äthyl)ester und Äthylcellulose] werden im Gewichtsverhältnis 2,5 : 1 bis 5 : 1, aber insbesondere im Verhältnis 3 : 1 verwendet.

Es ist vorteilhaft, den fertig umhüllten Körnern geringe Mengen, z. B. von 0,5 bis 1% kolloidales Siliciumdioxid, z. B. das durch Degussa, Frankfurt (BRD) vertriebene Aerosil® zuzumischen. Durch diesen Zusatz wird die Freifließfähigkeit der Körner noch verbessert. Weiter können natürlich dem Hüllstoffgemisch auch sonstige Hilfsstoffe in geringen Mengen, wie z. B. Farbstoffe oder Aromastoffe zugesetzt werden.

Die Herstellung der erfindungsgemäß verwendeten Arzneimittelkörner kann in an sich bekannter Weise erfolgen. Dies gilt auch für die umhüllten Körner. Diese werden in den für diesen Zweck bekannten Wirbelschicht-Sprühapparaten oder in Dragierkesseln hergestellt. Das Hüllstoffgemisch wird als wäßrige Dispersioin bei Raumtemperatur zugeführt. Das Versprühen erfolgt am besten mit Luft von einer Temperatur von 25 bis 30°C. Auf diese Weise werden ohne weiteres die einzelnen Körner erhalten, d. h. eine unerwünschte Agglomeration zu Granulaten erfolgt nicht.

Überraschenderweise lassen sich die erfindungsgemäß verwendeten Arzneimittelkörner zusammen mit einem Sprengmittel mit hoher Sprengkraft und Bindemitteleigenschaften und mit den sonst für die Tablettierung üblichen Hilfsstoffen innerhalb eines weiten Dosisbereiches leicht zu Formkörpern, wie z. B. Tabletten oder kapsel- oder stäbchenförmigen Preßlingen verpressen. Die so hergestellten Formkörper haben die Eigenschaft, daß sie bereits schon im Magen des so Behandelten rasch wieder in Einzelkörner zerfallen und sich somit gut verteilen. Auf diese Weise wird eine örtliche Überkonzentration des Wirkstoffes im Verdauungstrakt verhindert und für eine gleichmäßige, langsam und über ein großes Resorptionsgebiet verteilte Wirkstoffabgabe gesorgt. Durch mikroskopische Untersuchung wurde festgestellt, daß die einzelnen Körner durch das Verpressen kaum beschädigt werden, so daß bei der Freisetzung des Wirkstoffes aus denselben diese ihre ursprünglichen vorteilhaften Eigenschaften praktisch voll entfalten können. Soll ein Preßling mit zwei oder mehreren Wirkstoffen hergestellt werden, so können individuell gefärbte Arzneistoffkörner separat hergestellt werden, was die Kennzeichnung verbessert und dann auch den Patienten darauf aufmerksam macht, daß er ein Medikament mit zwei oder mehreren Wirkstoffen vor sich hat.

Als Sprengmittel mit hoher Sprengkraft und Bindemitteleigenschaften für die erfindungsgemäß herstellbaren Preßlinge verwendet man quervernetztes Polyvinylpolypyrrolidon (PVPP), wie z. B. das durch die GAF Corporation, New York, N. Y. (USA) vertriebene Polyplasdone® XL bzw. Kollidon® CL (BASF, Ludwigshafen/Rhein, BRD).

Bei den sonst noch für die Tablettierung üblichen Hilfsstoffen handelt es sich vor allem um Binde- und Schmier- bzw. Antiklebmittel.

Zur Herstellung der erfindungsgemäß herstellbaren Preßlinge können die üblichen Tablettenpressen verwendet werden.

Da die mechanische Festigkeit der Preßlinge überraschend gut ist, lassen sich auch alle gewünschten üblichen Formen, wie z. B. Tabletten, kapsel- oder stäbchenförmige Preßlinge mit oder ohne Bruchkerben herstellen. Diese Preßlinge lassen sich gewünschtenfalls auch mit einer für diesen Zweck bekannten Schutzlackierung versehen.

Als erfindungsgemäß zu verarbeitende Arzneimittelwirkstoffe von Granulatform bzw. Kristallen geeigneter Größe eignen sich im Prinzip alle, die für eine perorale Abgabe geeignet sind und für die eine verzögerte Abgabe im Magen-Darmtrakt erwünscht ist. Besonders vorteilhaft ist die vorliegende Erfindung jedoch bei Verwendung von Wirkstoffen, die bei höherer Konzentration lokale Reizungen der Schleimhaut im Magen-Darmtrakt verursachen können, und die in großen Einzeldosen verabreicht werden. Dies trifft z. B. für Kaliumchlorid zu, das bei Kaliummangelzuständen verabreicht wird, oder für Lithiumsalze in der Psychotherapie.

Beispiel 1

| Zusammensetzung | pro Dosis | pro Ansatz |
|---|---|---|
| Kaliumchloridkristalle mit Teilchengröße von 0,5—1,2 mm | 1000,0 mg | 5000,0 g |
| Eudragit® E30D Feststoff | 180,0 mg | 900,0 g |
| Aquacoat® ECD Feststoff | 55,0 mg | 275,0 g |
| Aerosil® 200 | 4,0 mg | 20,0 g |
| Aerosil® 200 | 6,0 mg | 30,0 g |
| Polyplasdone® XL | 150,0 mg | 750,0 g |
| Magnesiumstearat | 5,0 mg | 25,0 g |
| | 1400,0 mg | 7000,0 g |

Herstellung

A) Umhüllte Körner

1. Kaliumchlorid vorlegen
2. Eudragit® E30D und Aquacoat® ECD30 unter schwachem Rühren mischen
3. 1. mit 2. besprühen im Wirbelschichtgranulator (Wirbelschichtgranulator Aeromatik ST 7)
   - im Gleichstrom sprühen
   - die Mischung der Dispersionen während des Sprühvorganges rühren
   - Zulufttemperatur 28° C
   - Durchsatz ca. 80 g/Minute
4. Trocknen bei 28° C Zulufttemperatur (Wirbelschichttrockner Aeromatik ST 7) ca. 10 Minuten
5. Trockene umhüllte Körner mit Aerosil® 200 während 10 Minuten mischen
6. Mischung 5. sieben, Sieb 1,5—2,0 mm.

Eigenschaften:

Freifließend, geschmacklos.
Freigabeeigenschaften von Kaliumchlorid (Van der Kamp Zerfallstester, in Wasser von 37° C)

| | |
|---|---|
| nach 1 Stunde | ca. 24% |
| nach 2 Stunden | ca. 45% |
| nach 4 Stunden | ca. 81% |
| nach 6 Stunden | ca. 96% |

B) Preßling

Umhüllte Körner, Aerosil® 200, Polyplasdone® XL und Magnesiumstearat während 15 Minuten mischen. Mischung auf handelsüblicher Tablettiermaschine zu Stäbchen verpressen (z. B. Korsch EKO); Stempelgröße 20,7 × 8,6 mm.

Eigenschaften:

Zerfall in Wasser von 37° C: < 5 Minuten
Freigabeeigenschaften von Kaliumchlorid (Van der Kamp Zerfallstester in Wasser von 37° C):

| | |
|---|---|
| nach 1 Stunde | ca. 30% |
| nach 2 Stunden | ca. 50% |
| nach 4 Stunden | ca. 80% |
| nach 6 Stunden | ca. 98% |

Beispiel 2

| Zusammensetzung | pro Dosis | pro Ansatz |
|---|---|---|
| Kaliumchloridkristalle mit Teilchengröße von 0,3—0,8 mm | 600,0 mg | 900,0 g |
| Eudragit® E30D Feststoff | 115,0 mg | 172,5 g |
| Aquacoat® ECD Feststoff | 35,0 mg | 52,5 g |
| Aerosil® 200 | 5,0 mg | 7,5 g |
| Polyplasdone® XL | 92,0 mg | 138,0 g |
| Magnesiumstearat | 3,0 mg | 4,5 g |
| | 850,0 mg | 1275,0 g |

Herstellung

A) Umhüllte Körner

1. Kaliumchlorid vorlegen
2. Eudragit® E30D und Aquacoat® ECD 30 unter schwachem Rühren mischen
3. 1. mit 2. besprühen im Wirbelschichtgranulator (Wirbelschichtgranulator Aeromatik Strea 1)
   — im Gleichstrom sprühen
   — die Mischung der Dispersionen während des Sprühvorganges rühren
   — Zulufttemperatur 28° C
   — Durchsatz ca. 8 g/Minute
4. Trocknen bei 28° C Zulufttemperatur (Wirbelschichttrockner Aeromatik ST 7) ca. 10 Minuten
5. Trockene umhüllte Körner mit Aerosil® 200 während 10 Minuten mischen
6. Mischung 5. sieben, Sieb 1,5—2,0 mm.

Eigenschaften:

Freifließend, geschmacklos.
Freigabeeigenschaften von Kaliumchlorid (Van der Kamp Zerfallstester, in Wasser von 37° C)

| | |
|---|---|
| nach 1 Stunde | ca. 38% |
| nach 2 Stunden | ca. 74% |
| nach 3 Stunden | ca. 96% |

B) Preßling

Umhüllte Körner, quervernetztes Polyvinylpolypyrrolidon und Magnesiumstearat während 15 Minuten mischen. Mischung auf handelsüblicher Tablettiermaschine zu Stäbchen verpressen (z. B. Korsch EKO); Stempelgröße 20,7 x 8,6 mm.

Eigenschaften:

Zerfall in Wasser von 37° C: <5 Minuten
Freigabeeigenschaften von Kaliumchlorid (Van der Kamp Zerfallstester in Wasser von 37° C):

| | |
|---|---|
| nach 1 Stunde | ca. 42% |
| nach 2 Stunden | ca. 70% |
| nach 3 Stunden | ca. 90% |

Beispiel 3

| Zusammensetzung | Dosis mg/Ansatz g |
|---|---|
| Pirprofenkristalle (0,5 mm) | 600,0 mg/g |
| Eudragit® E30D (Feststoff) | 24,0 mg/g (80 g Dispersion) |
| Aquacoat® ECD30 (Feststoff) | 6,0 mg/g (20 g Dispersion) |
| Aerosil® 200 | 5,0 mg/g |
| Polyplasdone® XL | 150,0 mg/g |
| Baumwollsamenöl hydr. | 5,0 mg/g |

Herstellung

A) Umhüllte Körner

1. Pirprofen vorlegen
2. Eudragit® E30D und Aquacoat® ECD30 unter schwachem Rühren vermischen
3. 1. mit 2. besprühen im Wirbelschichtgranulator Aeromatik Strea 1:
   — im Gleichstrom sprühen
   — Dispersion rühren
   — Zulufttemperatur 35° C
   — Durchsatz ca. 8 g/Minute
4. Trocknen bei 35° C Zulufttemperatur (Wirbelschichttrockner Strea 1) ca. 10 Minuten
5. Trockene umhüllte Körner mit Aerosil® 200 während 10 Minuten mischen
6. Mischung 5. durch Sieb 2,0 mm sieben.

B) Preßlinge

— 5. mit Polyplasdone® XL und hydriertem Baumwollsamenöl 15 Minuten im Taumelmischer Typ Turbula® mischen
— Homogene Mischung 6. auf einer Tablettenpresse EKO zu stäbchenförmigen Tabletten 16,4 x 8,6 mm verpressen.

Prüfungsdaten:

| | | |
|---|---|---|
| Zerfall in Wasser (37° C) | in Einzelpartikel | 30 Sek. |
| Zerfall in Magensaft (37° C) | in Einzelpartikel | 1 Min. |

Beispiel 4

| Zusammensetzung | Dosis mg/Ansatz g |
|---|---|
| Zerolit®225 4% quervernetzt (Teilchengröße 0,1—0,3 mm) 200 mg/g beladen mit 100 mg/g Pirprofen, Gesamtgewicht | 300 mg/g |
| Polyplasdone® XL | 150 mg/g |
| Avicel®PH102 | 150 mg/g |
| | 600 mg/g |

Herstellung

Zerolit® 225 (kationischer Ionenaustauscher, von Dia-proxim Ltd., England), Polyplasdone® XL und Avicel® PH102 (granulierte mikrokristalline Cellulose von FMC, USA) durch Sieb mit 0,8 mm Maschenweite sieben.

Die gesiebten Komponenten werden 10 Minuten in einem Turbula Mischer gemischt.

Die homogene Mischung wird auf einer Tablettiermaschine, z. B. Korsch EKO, zu runden Tabletten verpreßt.

Eigenschaften:

| | |
|---|---|
| Ø Tabletten | 9,0 mm |
| Härte Tabletten | ca. 80 Newton |
| Zerfall (Wasser 37° C) | ca. 15 Sekunden. |

**Patentansprüche für die Vertragsstaaten BE, CH, DE, FR, IT, LI, LU, NL, SE**

1. Schnellzerfallende Arzneimittel-Preßlinge, die im wesentlichen aus einem verpreßten Gemisch von

a) einem Arzneimittel in körniger Retardform, bestehend aus einem granulierten oder kristallinen Arzneimittelwirkstoff, der umhüllt ist mit einem Hüllstoff, der im wesentlichen aus einem homogenen Gemisch eines wasserlöslichen oder in Wasser dispergierbaren Poly(H+meth)-acrylsäure(methyl+äthyl)esters und einer in Wasser unlöslichen, aber in Wasser dispergierbaren Äthylcellulose besteht, wobei das Gewichtsverhältnis 2,5 : 1 bis 5 : 1 beträgt, und

b) quervernetztem Polyvinylpyrrolidon als Sprengmittel mit hoher Sprengkraft sowie Bindemitteleigenschaften

bestehen.

2. Schnellzerfallende Arzneimittel-Preßlinge nach Anspruch 1, dadurch gekennzeichnet, daß das Mischverhältnis der Hüllstoffe 3 : 1 beträgt.

3. Schnellzerfallende Arzneimittel-Preßlinge nach Anspruch 1, dadurch gekennzeichnet, daß das Hüllstoffgemisch zusätzlich noch kolloidales Siliziumdioxid enthält.

4. Schnellzerfallende Arzneimittel-Preßlinge nach Anspruch 1, dadurch gekennzeichnet, daß der Arzneimittelwirkstoff Kaliumchlorid im Größenbereich 0,3—1,2 mm Durchmesser ist.

5. Schnellzerfallende Arzneimittel-Preßlinge nach Anspruch 1, dadurch gekennzeichnet, daß der Arzneimittelwirkstoff Kaliumchlorid im Größenbereich 0,5—1,2 mm Durchmesser ist.

6. Schnellzerfallende Arzneimittel-Preßlinge nach Anspruch 1, dadurch gekennzeichnet, daß der Arzneimittelwirkstoff Pirprofen im Größenbereich von 0,5—1,2 mm Durchmesser ist.

7. Schnellzerfallende Arzneimittel-Preßlinge nach Anspruch 1, dadurch gekennzeichnet, daß der Arzneimittelwirkstoff Diclofenac-Natrium im Größenbereich von 0,1—0,3 mm Durchmesser ist.

**Patentansprüche für den Vertragsstaat AT**

1. Verfahren zur Herstellung von schnellzerfallenden Arzneimittel-Preßlingen, dadurch gekennzeichnet, daß man ein Gemisch, das im wesentlichen aus

a) einem Arzneimittel in körniger Retardform bestehend aus einem granulierten oder kristallinen Arzneimittelwirkstoff, der umhüllt ist mit einem Hüllstoff, der im wesentlichen aus einem homogenen Gemisch eines wasserlöslichen oder in Wasser dispergierbaren Poly(H+meth)-acrylsäure(methyl+äthyl)esters und einer in Wasser unlöslichen, aber in Wasser dispergierbaren Äthylcellulose besteht, wobei das Gewichtsverhältnis 2,5 : 1 bis 5 : 1 beträgt, und

b) quervernetztem Polyvinylpyrrolidon als Sprengmittel mit hoher Sprengkraft sowie Bindemitteleigenschaften

besteht, verpreßt.

2. Verfahren nach Anspruch 1, dadurch gekennzeichnet, daß das Mischungsverhältnis der Hüllstoffe 3 : 1 beträgt.

3. Verfahren nach Anspruch 1, dadurch gekennzeichnet, daß das Hüllstoffgemisch zusätzlich noch kolloidales Siliziumdioxid enthält.

4. Verfahren nach Anspruch 1, dadurch gekennzeichnet, daß der Arzneimittelwirkstoff Kaliumchlorid im Größenbereich 0,2—1,2 mm Durchmesser ist.

5. Verfahren nach Anspruch 1, dadurch gekennzeichnet, daß der Arzneimittelwirkstoff Kaliumchlorid im Größenbereich 0,5—1,2 mm Durchmesser ist.

6. Verfahren nach Anspruch 1, dadurch gekennzeichnet, daß der Arzneimittelwirkstoff Pirprofen im Größenbereich von 0,5—1,2 mm Durchmesser ist.

7. Verfahren nach Anspruch 1, dadurch gekennzeichnet, daß der Arzneimittelwirkstoff Diclofenac-Natrium im Größenbereich von 0,1—0,3 mm Durchmesser ist.

**Claims for the Contracting States: DE, FR, CH, LI, IT, NL, BE, SE, LU**

1. A rapid-disintegrating moulded drug formulation consisting substantially of a compressed mixture of

a) a drug in granular delayed-release form comprising a granular or cristalline pharmaceutical active ingredient which is coated with a coating composition substantially of a homogenous mixture of the water-soluble or water-dispersible methyl+ethyl esters of poly(H+meth)acrylic acid and a water-insoluble but water-dispersible ethyl cellulose, the weight ratio being 2.5 : 1 to 5 : 1, and
b) a crosslinked polyvinyl pyrrolidone as disintegrator with excellent disintegrating properties as well as binder properties.

2. A rapid-disintegrating moulded drug formulation according to claim 1, wherein the ratio of the components of the coating composition is 3 : 1.

3. A rapid-disintegrating moulded drug formulation according to claim 1, wherein the coating composition additionally contains colloidal silica.

4. A rapid-disintegrating moulded drug formulation according to claim 1, wherein the pharmaceutical active ingredient is potassium chloride having a diameter in the range from 0.3 to 1.2 mm.

5. A rapid-disintegrating moulded drug formulation according to claim 1, wherein the pharmaceutical active ingredient is potassium chloride having a diameter in the range from 0.5 to 1.2 mm.

6. A rapid-disintegrating moulded drug formulation according to claim 1, wherein the pharmaceutical active ingredient is pirprofen having a diameter in the range from 0.5 to 1.2 mm.

7. A rapid-disintegrating moulded drug formulation according to claim 1, wherein the pharmaceutical active ingredient is diclofenac-sodium having a diameter in the range from 0.1 to 0.3 mm.

**Claims for the Contracting State: AT**

1. A process for the preparation of a rapid-distintegrating moulded drug formulation, which process comprises compressing a mixture consisting substantially of

a) a drug in granular delayed-release form comprising a granular or cristalline pharmaceutical active ingredient which is coated with a coating composition consisting substantially of a homogenous mixture of the water-soluble or water-dispersible methyl+ethyl esters of poly(H+meth)acrylic acid and a water-insoluble but water-dispersible ethyl cellulose, the weight ratio being 2.5 : 1 to 5 : 1, and
b) a crosslinked polyvinyl pyrrolidone as disintegrator with excellent disintegrating properties as well as binder properties.

2. A process according to claim 1, wherein the ratio of the components of the coating composition is 3 : 1.

3. A process according to claim 1, wherein the coating composition additionally contains colloidal silica.

4. A process according to claim 1, wherein the pharmaceutical active ingredient is potassium chloride having a diameter in the range from 0.3 to 1.2 mm.

5. A process according to claim 1, wherein the pharmaceutical active ingredient is potassium chloride having a diameter in the range from 0.5 to 1.2 mm.

6. A process according to claim 1, wherein the pharmaceutical active ingredient is pirprofen having a diameter in the range from 0.5 to 1.2 mm

7. A process according to claim 1, wherein the pharmaceutical active ingredient is diclofenac-sodium having a diameter in the range from 0.1 to 0.3 mm.

**Revendications pour les Etats contractants: DE, FR, CH, LI, IT, NL, BE, SE, LU**

1. Comprimés pharmaceutiques à désagrégation rapide, qui se composent pour l'essentiel d'un mélange comprimé de

a) un médicament sous forme retard en grain, constitué d'une substance active médicamenteuse granulée ou cristalline, qui est enrobée d'un produit d'enrobage constitué pour l'essentiel d'un mélange homogène d'un (méthyl+éthyl)-ester d'acide poly(H+méth)-acrylique soluble dans l'eau ou dispersable dans l'eau et d'une éthylcellulose insoluble dans l'eau mais dispersable dans l'eau, où le rapport pondéral s'élève à 2,5 : 1 à 5 : 1, et
b) de la polyvinylpyrrolidone réticulée comme solvant, avec un fort pouvoir solvant ainsi que des propriétés de liant.

2. Comprimés pharmaceutiques à désagrégation rapide selon la revendication 1, caractérisés en ce que le rapport de mélange des produits d'enrobage s'élève à 3 : 1.

3. Comprimés pharmaceutiques à désagrégation rapide selon la revendication 1, caractérisés en ce que le mélange d'enrobage contient encore en outre du dioxyde de silicium colloïdal.

4. Comprimés pharmaceutiques à désagrégation rapide selon la revendication 1, caractérisés en ce que la substance active médicamenteuse est du chlorure de potassium dans un intervalle de taille de 0,3—1,2 mm de diamètre.

5. Comprimés pharmaceutiques à désagrégation rapide selon la revendication 1, caractérisés en ce que la substance active médicamenteuse est du chlorure de potassium dans un intervalle de taille de 0,5—1,2 mm de diamètre.

6. Comprimés pharmaceutiques à désagrégation rapide selon la revendication 1, caractérisés en ce que la substance active médicamenteuse est le pirprofène dans un intervalle de taille de 0,5—1,2 mm de diamètre.

7. Comprimés pharmaceutiques à désagrégation rapide selon la revendication 1, caractérisés en ce que la substance active médicamenteuse est le diclofénac sodique dans un intervalle de taille de 0,1—0,3 mm de diamètre.

**Revendications pour l'Etat contractant: AT**

1. Procédé de préparation de comprimés pharmaceutiques à désagrégation rapide, caractérisé en ce qu'on comprime un mélange constitué pour l'essentiel de

a) un médicament sous forme retard en grains constitué d'une substance active médicamenteuse granulée ou cristalline, qui est enrobée d'un produit d'enrobage qui se compose pour l'essentiel d'un mélange homogène d'un (méthyl+éthyl)-ester d'acide poly(H+méth)-acrylique soluble dans l'eau ou dispersable dans l'eau et d'une éthylcellulose insoluble dans l'eau mais dispersable dans l'eau, où le rapport pondéral s'élève à 2,5 : 1 à 5 : 1, et
b) de la polyvinylpyrrolidone réticulée comme solvant avec un fort pouvoir solvant ainsi que des propriétés de liant.

2. Procédé selon la revendication 1, caractérisé en ce que le rapport de mélange des produits d'enrobage s'élève à 3 : 1.

3. Procédé selon la revendication 1, caractérisé en ce que le mélange de produits d'enrobage contient encore en outre du dioxyde de silicium colloïdal.

4. Procédé selon la revendication 1, caractérisé en ce que la substance active médicamenteuse est du chlorure de potassium dans un intervalle de taille de 0,2—1,2 mm de diamètre.

5. Procédé selon la revendication 1, caractérisé en ce que la substance active médicamenteuse est du chlorure de potassium dans un intervalle de taille de 0,5—1,2 mm de diamètre.

6. Procédé selon la revendication 1, caractérisé en ce que la substance active médicamenteuse est le pirprofène dans un intervalle de taille de 0,5—1,2 mm de diamètre.

7. Procédé selon la revendication 1, caractérisé en ce que la substance active médicamenteuse est le diclofénac sodique dans un intervalle de taille de 0,1—0,3 mm de diamètre.